# EUROPÄISCHE PATENTSCHRIFT

(11) **EP 3 351 236 B1**
(45) Veröffentlichungstag und Bekanntmachung des Hinweises auf die Patenterteilung: **13.05.2020**
(21) Anmeldenummer: 18150549.6
(22) Anmeldetag: 08.01.2018
(51) Int. Cl.: A61K 8/39, A61K 8/41, A61K 8/35, A61K 8/49, A61K 8/37, A61Q 17/04

(54) **OCTOCYLENFREIES SONNENSCHUTZMITTEL MIT POLYGLYCERYL-10 STEARAT**
OCTOCYLENE-FREE SUNSCREEN WITH POLYGLYCERYL-10 STEARATE
PRÉPARATION ANTI-SOLAIRE SANS OCTOCYLÈNE À BASE DE STÉARATE DE POLYGLYCÉRYLE-10

(30) Priorität: 18.01.2017 DE 102017200723
(43) Veröffentlichungstag der Anmeldung: 25.07.2018
(73) Patentinhaber: Beiersdorf AG, 20253 Hamburg (DE)
(72) Erfinder: Schade, Tatjana, 25866 Mildstedt/Rosendahl (DE); von der Fecht, Stephanie, 22880 Wedel (DE); Schuldt, Lisa, 22547 Hamburg (DE); Sprock, Sarah, 22297 Hamburg (DE); Borchers, Kathrin, 21614 Buxtehude (DE)

(56) Entgegenhaltungen:
- DE-A1-102007 024 346
- DE-A1-102013 213 174
- US-A1- 2014 308 220

## Beschreibung

Die vorliegende Erfindung betrifft eine kosmetische Zubereitung enthaltend eine UV-Filterkombination aus
a) Hexyl 2-[4-(diethylamino)-2-hydroxybenzoyl]benzoate (INCI: Diethylamino hydroxybenzoyl hexyl benzoate),
b) 4-(tert.-Butyl)-4'-methoxydibenzoylmethan (INCI Butyl Methoxydibenzoylmethane)
c) Polyglycerylfettsäureester, wobei die Zubereitung frei ist von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen), Verfahren zur Reduzierung der durch UV-Lichtschutzfilter enthaltenden kosmetischen Zubereitungen hervorgerufenen Textilverfleckung, dadurch gekennzeichnet, dass dem Kosmetikum Polyglycerylfettsäureester als Emulgator enthält und Verwendung von Polyglycerylfettsäureester in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Sonnenschutzmittel haben das Problem, dass eine Vielzahl von UV-Filtern in den Zubereitungen nicht besonders gut löslich ist. Insbesondere wenn Zubereitungen mit hohem Lichtschutzfaktor und hohem UV-Filtergehalt entwickelt werden, stellt die Löslichkeit von 4-(tert.-Butyl)-4'-methoxydibenzoylmethan (INCI Butyl Methoxydibenzoylmethane) und Triazinderivaten ein Problem für die Entwickler dar. Um dieses Problem zu lösen wurde in der Vergangenheit der flüssige UV-B-Filter Octocrylen als UV-Filter und Lösungsmittel verwendet. Octocrylen dient darüber hinaus der Photostabilisierung von 4-(tert.-Butyl)-4'-methoxydiben-zoylmethan.

Der Nachteil des Standes der Technik besteht nun in dem Umstand, dass der Einsatz von Octocrylen, trotz der Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass dieser UV-Filter möglicherweise hormonell wirksam sein könnte. Auch wenn, trotz des jahrzehntelangen weltweiten Einsatzes dieses UV-Filters in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen und ein Sonnenschutzmittel mit hohem Lichtschutzfaktor zu entwickeln, bei dem die UV-Filter (insbesondere 4-(tert.-Butyl)-4'-methoxydibenzoylmethan und Triazinderivate, insbesondere 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und 2,4,6-Tris-[anilino-(p-carbo-2'-ethyl-1'-hexyloxy)]-1,3,5-triazin (INCI: Ethylhexyl Triazone)) stabil gelöst werden, ohne dass Octocrylen als Lösungsmittel und Stabilisator eingesetzt wird.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) zu entwickeln, die nicht-wasserlösliche UV-A-Filter wie 4-(tert.-Butyl)-4'-methoxydibenzoylmethan (INCI Butyl Methoxydibenzoylmethane) und (2-[-4-(Diethylamino)-2-hydroxybenzoyl] Benzoesäurehexylester (INCI: Diethylamino Hydroxybenzoyl Hexyl Benzoate) sowie gegebenenfalls Breitbandfilter wie Bis-Ethylhexyloxyphenol Methoxyphenyl Triazine enthält, welche sich leichter aus den mit der Zubereitung kontaminierten Textilen herauswaschen lassen.

Überraschend gelöst werden die Aufgaben durch eine kosmetische Zubereitung enthaltend eine UV-Filterkombination aus
a) Hexyl 2-[4-(diethylamino)-2-hydroxybenzoyl]benzoate (INCI: Diethylamino hydroxybenzoyl hexyl benzoate),
b) 4-(tert.-Butyl)-4'-methoxydibenzoylmethan (INCI Butyl Methoxydibenzoylmethane)
c) Polyglycerylfettsäureester, wobei die Zubereitung frei ist von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenyl-acrylat (INCI: Octocrylen), dadurch gekennzeichnet, dass als Polyglycerylfettsäureester Polyglyceryl-10 Stearat (INCI Polyglyceryl-10 Stearate) eingesetzt wird.

Überraschend gelöst werden die Aufgaben ferner durch ein Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche diese UV-A Filter und/oder Breitbandfilter enthält, kontaminiert sind, dadurch gekennzeichnet, dass der kosmetischen Zubereitung Polyglycerylfettsäureester zugesetzt wird, sowie durch ein
Verfahren zur Reduzierung der durch UV-Lichtschutzfilter enthaltenden kosmetischen Zubereitungen hervorgerufenen Textilverfleckung, dadurch gekennzeichnet, dass dem Kosmetikum Polyglycerylfettsäureester als Emulgator enthält.

Nicht zuletzt werden die Aufgaben gelöst durch die Verwendung von Polyglycerylfettsäureester in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

Zwar kennt der Stand der Technik die DE 102014216602, DE 102014202956, DE 102013213170, DE 102013200819 sowie die DE 102011088962, doch konnten diese Schriften nicht den Weg zur vorliegenden Erfindung weisen.

Ferner kennt der Fachmann die DE102007024346, DE 102013213174 und US 2014/0308220, die ebenfalls nicht den Weg zur vorliegenden Erfindung weisen konnten.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Der erfindungsgemäß besonders bevorzugte Polyglycerylfettsäureester ist im Hinblick auf die erfindungsgemäßen Verfahren und die erfindungsgemäße. Verwendung Polyglyceryl-10 Stearat (INCI Polyglyceryl-10 Stearate).

Es ist erfindungsgemäß vorteilhaft, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und/oder 2,4,6-Tris-[anilino-(p-carbo-2'-ethyl-1'-hexyloxy)]-1,3,5-triazin (INCI: Ethylhexyl Triazone) enthält.

Dabei ist es erfindungsgemäß bevorzugt, wenn die Zubereitung eine Kombination aus Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und 2,4,6-Tris-[anilino-(p-carbo-2'-ethyl-1'-hexyloxy)]-1,3,5-triazin (INCI: Ethylhexyl Triazone) enthält.

Erfindungsgemäß vorteilhafte Ausführungsformen der vorliegenden Erfindung sind dadurch gekennzeichnet, dass die Zubereitung 2-Phenylbenzimidazol-5-sulfonsäuresalze und/oder 2-Ethylhexyl 2-hydroxybenzoat (INCI: Ethylhexyl Salicylate) enthält.

Erfindungsgemäß bevorzugte Ausführungsformen der vorliegenden Erfindung sind dadurch gekennzeichnet, dass die Zubereitung 2-Phenylbenzimidazol-5-sulfonsäuresalze und 2-Ethylhexyl 2-hydroxybenzoat (INCI: Ethylhexyl Salicylate) enthält.

Es ist vorteilhaft im Sinne der vorliegenden Erfindung, wenn die Zubereitung Hexyl 2-[4-(diethylamino)-2-hydroxybenzoyl]benzoate (INCI: Diethylamino hydroxybenzoyl hexyl benzoate) in einer Konzentration von 0,05 bis1,5 Gewichts-%, bezogen auf das Gesamtgewicht der Zubereitung, enthält.

Es ist bevorzugt im Sinne der vorliegenden Erfindung, wenn die Zubereitung Hexyl 2-[4-(diethylamino)-2-hydroxybenzoyl]benzoate (INCI: Diethylamino hydroxybenzoyl hexyl benzoate) in einer Konzentration von 0,05 bis 1,0 Gewichts-%, bezogen auf das Gesamtgewicht der Zubereitung, enthält.

Es ist erfindungsgemäß vorteilhaft, wenn die Zubereitung 4-(tert.-Butyl)-4'-methoxydibenzoylmethan (INCI Butyl Methoxydibenzoylmethane) in einer Konzentration von 1,00 bis 5,00 Gewichts-%, bezogen auf das Gesamtgewicht der Zubereitung, enthält.

Es ist erfindungsgemäß bevorzugt, wenn die Zubereitung 4-(tert.-Butyl)-4'-methoxydibenzoylmethan (INCI Butyl Methoxydibenzoylmethane) in einer Konzentration von 1,25 bis 4,75 Gewichts-%, bezogen auf das Gesamtgewicht der Zubereitung, enthält.

Um erfindungsgemäß besonders vorteilhafte Resultate im Hinblick auf die Auswaschbarkeit der erfindungsgemäßen Zubereitung aus Textilien zu erzielen, sollte das Gewichtsverhältnis von Diethylamino hydroxybenzoyl hexyl benzoate zu Butyl Methoxydibenzoylmethane von 0,2:1 bis 1:5 betragen.

Erfindungsgemäß vorteilhafte Ausführungsformen der vorliegenden Erfindung sind dadurch gekennzeichnet, dass die Zubereitung Polyglyceryl-10 Stearat (INCI Polyglyceryl-10 Stearate) in einer Konzentration von 0,05 bis 1,25 Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung Polyglyceryl-10 Stearat (INCI Polyglyceryl-10 Stearate) in einer Konzentration von 0,5 bis 1,00 Gewichts-%, bezogen auf das Gesamtgewicht der Zubereitung, enthält.

Es ist vorteilhaft im Sinne der vorliegenden Erfindung, wenn die Gesamtkonzentration an 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und 2,4,6-Tris-[anilino-(p-carbo-2'-ethyl-1'-hexyloxy)]-1,3,5-triazin (INCI: Ethylhexyl Triazone) in der Zubereitung von 0,1 bis 4,5 Gewichts-%, bezogen auf das Gesamtgewicht der Zubereitung beträgt.

Es sind dabei drei erfindungsgemäße Fallkonstellationen zu unterscheiden:
Enthält die erfindungsgemäße Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und kein 2,4,6-Tris-[anilino-(p-carbo-2'-ethyl-1'-hexyloxy)]-1,3,5-triazin (INCI: Ethylhexyl Triazone), so wird das Bis-Ethylhexyloxyphenol methoxyphenyl Triazine in der Zubereitung in einer Konzentration von 0,05 bis 4,00 Gewichts-%, bezogen auf das Gesamtgewicht der Zubereitung erfindungsgemäß vorteilhaft eingesetzt..

Enthält die erfindungsgemäße Zubereitung kein 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) aber 2,4,6-Tris-[anilino-(p-carbo-2'-ethyl-1'-hexyloxy)]-1,3,5-triazin (INCI: Ethylhexyl Triazone), so wird das Ethylhexyl Triazone in der Zubereitung in einer Konzentration von 0,1 bis 4,5 Gewichts-%, bezogen auf das Gesamtgewicht der Zubereitung erfindungsgemäß vorteilhaft eingesetzt..

Enthält die erfindungsgemäße Zubereitung sowohl 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) als auch 2,4,6-Tris-[anilino-(p-carbo-2'-ethyl-1'-hexyloxy)]-1,3,5-triazin (INCI: Ethylhexyl Triazone), so wird das Bis-Ethylhexyloxyphenol methoxyphenyl Triazine in der Zubereitung in einer Konzentration von 0,05 bis 4,00 Gewichts-% und das Ethylhexyl Triazone in der Zubereitung in einer Konzentration von 0,1 bis 4,5 Gewichts-% jeweils bezogen auf das Gesamtgewicht der Zubereitung erfindungsgemäß vorteilhaft eingesetzt. Diese Kombination ist erfindungsgemäß besonders bevorzugt.

Erfindungsgemäß vorteilhafte Ausführungsformen der vorliegenden Erfindung sind dadurch gekennzeichnet, dass die Gesamtkonzentration an 2-Phenylbenzimidazol-5-sulfonsäuresalze und 2-Ethylhexyl 2-hydroxybenzoat (INCI: Ethylhexyl Salicylate) in der Zubereitung von 0,05 bis 5,00 Gewichts-%, bezogen auf das Gesamtgewicht der Zubereitung beträgt.

Dabei sind erfindungsgemäß drei Fallkonstellationen zu unterscheiden:
Enthält die erfindungsgemäße Zubereitung 2-Phenylbenzimidazol-5-sulfonsäuresalze und kein 2-Ethylhexyl 2-hydroxybenzoat (INCI: Ethylhexyl Salicylate), so ist es erfindungsgemäß vorteilhaft, diese Verbindung in einer Konzentration von 0,1 bis 1,5 Gewichts-%, bezogen auf das Gesamtgewicht der Zubereitung einzusetzen.

Enthält die erfindungsgemäße Zubereitung keine 2-Phenylbenzimidazol-5-sulfonsäuresalze, dafür jedoch 2-Ethylhexyl 2-hydroxybenzoat (INCI: Ethylhexyl Salicylate), so ist es erfindungsgemäß vorteilhaft, diese Verbindung in einer Konzentration von 0,05 bis 5,00 Gewichts-%, bezogen auf das Gesamtgewicht der Zubereitung einzusetzen.

Enthält die erfindungsgemäße Zubereitung sowohl 2-Phenylbenzimidazol-5-sulfonsäuresalze als auch 2-Ethylhexyl 2-hydroxybenzoat (INCI: Ethylhexyl Salicylate), so ist es erfindungsgemäß vorteilhaft, 2-Phenylbenzimidazol-5-sulfonsäuresalze in einer Konzentration von 0,1 bis 3,00 Gewichts-%, bezogen auf das Gesamtgewicht der Zubereitung einzusetzen und die 2-Ethylhexyl 2-hydroxybenzoat (INCI: Ethylhexyl Salicylate) sulfonsäuresalze in einer Konzentration von 0,05 bis 5,00 Gewichts-%, bezogen auf das Gesamtgewicht der Zubereitung einzusetzen. Diese Kombination ist erfindungsgemäß besonders bevorzugt.

Daraus ergibt sich als erfindungsgemäß ganz besonders bevorzugt eine Kombination aus
a) Hexyl 2-[4-(diethylamino)-2-hydroxybenzoyl]benzoate (INCI: Diethylamino hydroxybenzoyl hexyl benzoate),
b) 4-(tert.-Butyl)-4'-methoxydibenzoylmethan (INCI Butyl Methoxydibenzoylmethane)
c) Polyglyceryl-10 Stearat (INCI Polyglyceryl-10 Stearate),
d) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine)
e) 2,4,6-Tris-[anilino-(p-carbo-2'-ethyl-1'-hexyloxy)]-1,3,5-triazin (INCI: Ethylhexyl Triazone),
f) 2-Phenylbenzimidazol-5-sulfonsäuresalze und
g) 2-Ethylhexyl 2-hydroxybenzoat (INCI: Ethylhexyl Salicylate)
wobei die Zubereitung frei ist von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen).

Dabei werden bevorzugt die oben angegebenen Konzentrationen eingesetzt.

Darüber hinaus ist es erfindungsgemäß von Vorteil, wenn die Zubereitung eine Kombination aus Phenoxyethanol und Tocopherylacetat beziehungsweise Tocopherol enthält.

Dabei wird erfindungsgemäß bevorzugt die Kombination aus Phenoxyethanol und Tocopherylacetat eingesetzt.

In einem solchen Fall ist es wiederum erfindungsgemäß vorteilhaft, wenn die Zubereitung von 0,1 bis 0,9 Gewichts-% Phenoxyethanol und von 0,05 bis 0,5 Gewichts-% Tocopherylacetat, jeweils bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung einen oder mehrere der Parfümstoffe Hexylsalicylat, Linaylacetat, 2-Isobutyl-4-hydroxy-4-methyltetrahydropyran, Adipinsäurediester, Methylheptenon, alpha-Isomethylionon, Butylphenylmethylpropioal, Coumarin, Hexylcinnamal, Limonen, Linalool, Diethylsuccinat, Hydroxyisohexyl 3-Cyclohexencarboxaldehyd, Diethylsuccinat, Menthyl PCA und Citronellylmethylcrotonat, Benzyl Benzoate, Alpha-Isomethylionon, Benzylalkohol, Benzylcinnamat, Benzylsalicylat, Citronellol, Eugenol, Geraniol enthält.

Erfindungsgemäß vorteilhafte Ausführungsformen sind ferner dadurch gekennzeichnet, dass die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Nicht zuletzt ist es erfindungsgemäß von Vorteil, wenn die Zubereitung keine Parabene, sowie kein Methylisothiazolinon, Chlormethylisothiazolinon und DMDM-Hydantoin enthält, also frei ist von diesen Inhaltsstoffen.

Die erfindungsgemäße Zubereitung stellt üblicherweise eine ÖI-in-Wasser-Emulsion (O/W-Emulsion) dar. Diese kann die für derartige Zubereitungen üblichen Inhaltsstoffe enthalten.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi, quervernetztes Acrylat/C10-C30 Alkylacrylat Polymer und/oder Vinylpyrrolidon/Hexadecen-Copolymer enthält.

Ein Gehalt an Glycerin von mindestens 5 Gewicht-%, bezogen auf das Gesamtgewicht der Zubereitung, ist erfindungsgemäß besonders vorteilhaft.

Darüber hinaus ist es erfindungsgemäß von besonderem Vorteil, wenn die erfindungsgemäße Zubereitung Ethanol enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Hyaluronsäure und/oder deren Salze und/oder Licochalcon A enthält.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 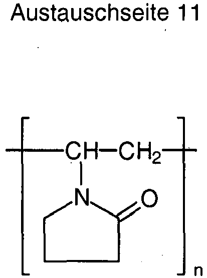 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

### Vergleichsversuch (Waschtest) und Beispiel

Mit dem folgenden Versuch konnte der erfindungsgemäße Effekt beispielhaft belegt werden: Es wurden verschiedene Emulgatorsysteme hergestellt. Auch ein System, welches 0,8% des erfindungsgemäßen Hilfsstoffes Polyglyceryl-10 Stearate enthält um die Verfleckungsreduzierende Wirkung (Reduktion b*) im Vergleich zu einer Formulierung ohne erfindungsgemäße Hilfsstoff mittels beschriebener Methode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1 °C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel,10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

Das nachfolgende Beispiel (Rezeptur 4) soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Tabelle 1: Getestete Formulierungen und deren Gelbwerte, db-Wert**

| | Beispiele [%] | | | |
|---|---|---|---|---|
| **INCI** | 1 | 2 | 3 | 4 |
| Sodium Stearoyl Glutamate | 0,30 | | | |
| Glyceryl Stearate SE | | | 1,00 | |
| Sodium Cetearyl Sulfate | | | 0,15 | |
| Glyceryl Stearate Citrate | | 2,00 | | |
| Polyglyceryl-10 Stearate | | | | 0,80 |
| Glyceryl Stearate | | | | 1,00 |
| Isopropyl Palmitate | 6,00 | 6,00 | 6,00 | 6,00 |
| Hydrogenated Coco-Glycerides | 1,00 | 1,00 | 1,00 | 1,00 |
| C18-38 Alkyl Hydroxystearoyl Stearate | 1,00 | | 0,50 | |
| Butylene Glycol Dicaprylate/Dicaprate | 2,00 | 3,00 | 2,00 | 2,00 |
| C12-15 Alkyl Benzoate | 5,00 | 5,00 | 5,00 | 5,00 |
| Silica Dimethyl Silylate | 1,00 | 1,00 | 1,00 | 1,00 |
| VP/Hexadecene Copolymer | 0,50 | 0,50 | 0,50 | 0,50 |
| Glycerin | 0,90 | 8,60 | 8,60 | 0,90 |
| Cetearyl Alcohol | 2,00 | 1,50 | 1,00 | 0,50 |
| Xanthan Gum | 0,40 | 0,40 | 0,40 | 0,40 |
| Acrylates/C10-30 Alkyl Acrylate Crosspolymer | 0,10 | 0,10 | 0,10 | 0,10 |
| Alcohol Denat. | 5,00 | 4,00 | 4,00 | 5,00 |
| Aqua + Trisodium EDTA | 1,00 | 1,00 | 1,00 | 1,00 |
| Butyl Methoxydibenzoylmethane | 3,00 | 3,00 | 3,00 | 3,00 |
| Phenylbenzimidazole Sulfonic Acid | 0,50 | 0,50 | 0,50 | 0,50 |
| Ethylhexyl Salicylate | 2,38 | 2,38 | 2,38 | 2,38 |
| Ethylhexyl Triazone | 1,50 | 1,50 | 1,50 | 1,50 |
| Bis-Ethylhexyloxyphenol Methoxyphenyl Triazine | 2,00 | 2,00 | 2,00 | 2,00 |
| Diethylamino Hydroxybenzoyl Hexyl Benzoate | 0,50 | 0,50 | 0,50 | 0,50 |
| Titanium Dioxide (nano) | 3,00 | 3,00 | 3,00 | 3,00 |
| Sodium Hydroxide | Add pH 7 | Add pH 7 | Add pH 7 | Add pH 7 |
| preservatives | add 100 | add 100 | add 100 | add 100 |
| Wasser | add 100 | add 100 | add 100 | add 100 |
| **Endwerte db** | **6,19** | **6,05** | **5,92** | **3,29** |

## Patentansprüche

1. Kosmetische Zubereitung enthaltend eine UV-Filterkombination aus
a) Hexyl 2-[4-(diethylamino)-2-hydroxybenzoyl]benzoate (INCI: Diethylamino hydroxybenzoyl hexyl benzoate),
b) 4-(tert.-Butyl)-4'-methoxydibenzoylmethan (INCI Butyl Methoxydibenzoylmethane)
c) Polyglycerylfettsäureester, wobei die Zubereitung frei ist von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen), **dadurch gekennzeichnet, dass** als Polyglycerylfettsäureester Polyglyceryl-10 Stearat (INCI Polyglyceryl-10 Stearate) eingesetzt wird.

2. Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche diese UV-A Filter und/oder Breitbandfilter enthält, kontaminiert sind, **dadurch gekennzeichnet, dass** der kosmetischen Zubereitung Polyglycerylfettsäureester, zugesetzt wird.

3. Verfahren zur Reduzierung der durch UV-Lichtschutzfilter enthaltenden kosmetischen Zubereitungen hervorgerufenen Textilverfleckung, **dadurch gekennzeichnet, dass** dem Kosmetikum Polyglycerylfettsäureester als Emulgator enthält.

4. Verwendung von Polyglycerylfettsäureester in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

5. Verfahren nach Anspruch 2 oder 3 oder Verwendung nach Anspruch 4, **dadurch gekennzeichnet, dass** als Polyglycerylfettsäureester Polyglyceryl-10 Stearat (INCI Polyglyceryl-10 Stearate) eingesetzt wird.

6. Zubereitung, Verfahren oder Verwendung nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und/oder 2,4,6-Tris-[anilino-(p-carbo-2'-ethy)-1'-hexyioxy)]-1,3,5-triazin (INCI: Ethylhexyl Triazone) enthält.

7. Zubereitung, Verfahren oder Verwendung nach einem der vorhergehenden Ansprüche, **dadurch gekennzeichnet, dass** die Zubereitung 2-Phenylbenzimidazol-5-sulfonsäuresalze und/oder 2-Ethylhexyl 2-hydroxybenzoat (INCI: Ethylhexyl Salicylate) enthält

8. Zubereitung, Verfahren oder Verwendung nach einem der vorhergehenden Ansprüche, **dadurch gekennzeichnet, dass** die Zubereitung Hexyl 2-[4-(diethylamino)-2-hydroxybenzoyl]benzoate (INCI: Diethylamino hydroxybenzoyl hexyl benzoate) in einer Konzentration von 0,05 bis 1,5 Gewichts-%, bezogen auf das Gesamtgewicht der Zubereitung, enthält.

9. Zubereitung, Verfahren oder Verwendung nach einem der vorhergehenden Ansprüche, **dadurch gekennzeichnet, dass** die Zubereitung 4-(tert.-Butyl)-4'-methoxy-dibenzoylmethan (INCI Butyl Methoxydibenzoylmethane) in einer Konzentration von 1,00 bis 5,00 Gewichts-%, bezogen auf das Gesamtgewicht der Zubereitung, enthält.

10. Zubereitung, Verfahren oder Verwendung nach einem der vorhergehenden Ansprüche, **dadurch gekennzeichnet, dass** das das Gewichtsverhältnis von Diethylamino hydroxybenzoyl hexyl benzoate zu Butyl Methoxydibenzoylmethane von 0,2:1 bis 1:5 beträgt.

11. Zubereitung, Verfahren oder Verwendung nach einem der vorhergehenden Ansprüche, **dadurch gekennzeichnet, dass** die Zubereitung Polyglyceryl-10 Stearat (INCI Polyglyceryl-10 Stearate) in einer Konzentration von 0,05 bis 1,25 Gewichts-%, bezogen auf das Gesamtgewicht der Zubereitung, enthält.

12. Zubereitung, Verfahren oder Verwendung nach einem der vorhergehenden Ansprüche, **dadurch gekennzeichnet, dass** die Gesamtkonzentration an 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) und 2,4,6-Tris-[anilino-(p-carbo-2'-ethyl-1'-hexyloxy)]-1,3,5-triazin (INCI: Ethylhexyl Triazone) in der Zubereitung von 0,05 bis 4,00 Gewichts-%, bezogen auf das Gesamtgewicht der Zubereitung beträgt.

13. Zubereitung, Verfahren oder Verwendung nach einem der vorhergehenden Ansprüche, **dadurch gekennzeichnet, dass** die Gesamtkonzentration an 2-Phenylbenzimidazol-5-sulfonsäuresalze und 2-Ethylhexyl 2-hydroxybenzoat (INCI: Ethylhexyl Salicylate) in der Zubereitung von 0,05 bis 5,00 Gewichts-%, bezogen auf das Gesamtgewicht der Zubereitung beträgt.

14. Zubereitung, Verfahren oder Verwendung nach einem der vorhergehenden Ansprüche, **dadurch gekennzeichnet, dass** die Zubereitung eine Kombination aus Phenoxyethanol und Tocopherylacetat beziehungsweise Tocopherol enthält.

15. Zubereitung, Verfahren oder Verwendung nach einem der vorhergehenden Ansprüche, **dadurch gekennzeichnet, dass** die Zubereitung einen oder mehrere der Parfümstoffe Hexylsalicylat, Linaylacetat, 2-lsobutyl-4-hydroxy-4-methyltetrahydropyran, Adipinsäurediester, Methylheptenon, alpha-Isomethylionon, Butylphenylmethylpropioal, Coumarin, Hexylcinnamal, Limonen, Linalool, Diethylsuccinat, Hydroxyisohexyl 3-Cyclohexencarboxaldehyd, Diethylsuccinat, Menthyl PCA und Citronellylmethylcrotonat, Benzyl Benzoate, Alpha-Isomethylionon, Benzylalkohol, Benzylcinnamat, Benzylsalicylat, Citronellol, Eugenol, Geraniol enthält.

16. Zubereitung, Verfahren oder Verwendung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

17. Zubereitung, Verfahren oder Verwendung nach einem der vorhergehenden Ansprüche, **dadurch gekennzeichnet, dass** die Zubereitung keine Parabene, sowie kein Methylisothiazolinon, Chlormethylisothiazolinon und DMDM-Hydantoin enthält, also frei ist von diesen Inhaltsstoffen.

## Claims

1. Cosmetic preparation comprising a UV filter combination of hexyl 2-[4-(diethylamino)-2-hydroxybenzoyl]benzoate (INCI: Diethylamino Hydroxybenzoyl Hexyl Benzoate),
4-(tert-butyl)-4'-methoxydibenzoylmethane (INCI Butyl Methoxydibenzoylmethane),
polyglyceryl fatty acid ester, wherein the preparation is free of 3-(4-methylbenzylidene)camphor, 2-hydroxy-4-methoxybenzophenone (INCI: Oxybenzone), 2-ethylhexyl 4-methoxycinnamate, isoamyl 4-methoxycinnamate and ethylhexyl 2-cyano-3,3-diphenylacrylate (INCI: Octocrylene), **characterized in that** polyglyceryl-10 stearate (INCI Polyglyceryl-10 Stearate) is used as polyglyceryl fatty acid ester.

2. Method for increasing the ability of organic, oil-soluble UVA filters and/or organic, oil-soluble broadband filters to be washed out from textiles which have been contaminated with a cosmetic preparation comprising these UVA filters and/or broadband filters, **characterized in that** polyglyceryl fatty acid ester is added to the cosmetic preparation.

3. Method for reducing the textile staining caused by cosmetic preparations comprising UV light protection filters, **characterized in that** the cosmetic comprises polyglyceryl fatty acid ester as emulsifier.

4. Use of polyglyceryl fatty acid ester in cosmetic preparations comprising UV light protection filters for facilitating the ability of the UV light protection filters to be washed out from textiles contaminated with the preparations.

5. Method according to Claim 2 or 3 or use according to Claim 4, **characterized in that** polyglyceryl-10 stearate (INCI Polyglyceryl-10 Stearate) is used as polyglyceryl fatty acid ester.

6. Preparation, method or use according to any of the preceding claims, **characterized in that** the preparation comprises 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and/or 2,4,6-tris[anilino-(p-carbo-2'-ethyl-1'-hexyloxy)]-1,3,5-triazine (INCI: Ethylhexyl Triazone).

7. Preparation, method or use according to any of the preceding claims, **characterized in that** the preparation comprises 2-phenylbenzimidazole-5-sulfonic acid salts and/or 2-ethylhexyl 2-hydroxybenzoate (INCI: Ethylhexyl Salicylate).

8. Preparation, method or use according to any of the preceding claims, **characterized in that** the preparation comprises hexyl 2-[4-(diethylamino)-2-hydroxybenzoyl]benzoate (INCI: Diethylamino Hydroxybenzoyl Hexyl Benzoate) at a concentration of 0.05 to 1.5% by weight, based on the total weight of the preparation.

9. Preparation, method or use according to any of the preceding claims, **characterized in that** the preparation comprises 4-(tert-butyl)-4'-methoxydibenzoylmethane (INCI Butyl Methoxydibenzoylmethane) at a concentration of 1.00 to 5.00% by weight, based on the total weight of the preparation.

10. Preparation, method or use according to any of the preceding claims, **characterized in that** the ratio by weight of diethylamino hydroxybenzoyl hexyl benzoate to butyl methoxydibenzoylmethane is from 0.2:1 to 1:5.

11. Preparation, method or use according to any of the preceding claims, **characterized in that** the preparation comprises polyglyceryl-10 stearate (INCI Polyglyceryl-10 Stearate) at a concentration of 0.05 to 1.25% by weight, based on the total weight of the preparation.

12. Preparation, method or use according to any of the preceding claims, **characterized in that** the total concentration of 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and 2,4,6-tris[anilino-(p-carbo-2'-ethyl-1'-hexyloxy)]-1,3,5-triazine (INCI: Ethylhexyl Triazone) in the preparation is from 0.05 to 4.00% by weight, based on the total weight of the preparation.

13. Preparation, method or use according to any of the preceding claims, **characterized in that** the total concentration of 2-phenylbenzimidazole-5-sulfonic acid salts and 2-ethylhexyl 2-hydroxybenzoate (INCI: Ethylhexyl Salicylate) in the preparation is from 0.05 to 5.00% by weight, based on the total weight of the preparation.

14. Preparation, method or use according to any of the preceding claims, **characterized in that** the preparation comprises a combination of phenoxyethanol and tocopheryl acetate or tocopherol.

15. Preparation, method or use according to any of the preceding claims, **characterized in that** the preparation comprises one or more of the perfumes hexyl salicylate, linalyl acetate, 2-isobutyl-4-hydroxy-4-methyltetrahydropyran, adipic acid diesters, methylheptenone, alpha-isomethyl ionone, butylphenylmethylpropional, coumarin, hexylcinnamal, limonene, linalool, diethyl succinate, hydroxyisohexyl 3-cyclohexenecarboxaldehyde, diethyl succinate, menthyl PCA and citronellyl methylcrotonate, benzyl benzoate, alpha-isomethyl ionone, benzyl alcohol, benzyl cinnamate, benzyl salicylate, citronellol, eugenol, geraniol.

16. Preparation, method or use according to any of the preceding claims, **characterized in that** the preparation comprises ethylhexylglycerin, propylene glycol, butylene glycol, 2-methylpropane-1,3-diol, 1,2-pentanediol, 1,2-hexanediol, 1,2-octanediol and/or 1,2-decanediol.

17. Preparation, method or use according to any of the preceding claims, **characterized in that** the preparation does not comprise any parabens and also no methylisothiazolinone, chloromethylisothiazolinone and DMDM hydantoin, i.e. is free of these ingredients.

## Revendications

1. Préparation cosmétique contenant une combinaison de filtres UV composée de
a) 2-[4-(diéthylamino)-2-hydroxybenzoyl]benzoate d'hexyle (INCI : Diethylamino hydroxybenzoyl hexyl benzoate),
b) 4-(tert-butyl)-4'-méthoxydibenzoylméthane (INCI Butyl Methoxydibenzoylmethane)
c) un ester d'acide gras de polyglycéryle, la préparation étant exempte de 3-(4-méthylbenzylidène)-camphre, de 2-hydroxy-4-méthoxybenzophénone (INCI : Oxybenzon), de 4-méthoxycinnamate de 2-éthylhexyle, de 4-méthoxycinnamate d'isoamyle et de 2-cyano-3,3-diphénylacrylate d'éthylhexyle (INCI : Octocrylen), **caractérisée en ce que** le stéarate de polyglycéryle-10 (INCI Polyglyceryl-10 Stearate) est utilisé en tant qu'ester d'acide gras de polyglycéryle.

2. Procédé pour l'augmentation de la lessivabilité de filtres UV-A organiques, solubles dans l'huile et/ou de filtres à large bande organiques, solubles dans l'huile en textiles, qui sont contaminés avec une préparation cosmétique qui contient ces filtres UV-A et/ou ces filtres à large bande, **caractérisé en ce qu'**un ester d'acide gras de polyglycéryle est ajouté à la préparation cosmétique.

3. Procédé pour la réduction de de la souillure des textiles provoquée par des préparations cosmétiques contenant des filtres protecteurs de lumière UV, **caractérisé en ce que** le produit cosmétique contient un ester d'acide gras de polyglycéryle en tant qu'émulsifiant.

4. Utilisation d'un ester d'acide gras de polyglycéryle dans des préparations cosmétiques contenant des filtres protecteurs de lumière UV pour la facilitation de la lessivabilité des filtres protecteurs de lumière UV en textiles contaminés avec les préparations.

5. Procédé selon la revendication 2 ou 3 ou utilisation selon la revendication 4, caractérisé(e) en ce que le stéarate de polyglycéryle-10 (INCI Polyglyceryl-10 Stearate) est utilisé en tant qu'ester d'acide gras de polyglycéryle.

6. Préparation, procédé ou utilisation selon l'une quelconque des revendications précédentes, caractérisé(e) en ce que la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine) et/ou de la 2,4,6-tris-[anilino-(p-carbo-2'-éthyl-1'-hexyloxy)]-1,3,5-triazine (INCI: Ethylhexyl Triazone).

7. Préparation, procédé ou utilisation selon l'une quelconque des revendications précédentes, caractérisé(e) en ce que la préparation contient des sels d'acide 2-phénylbenzimidazole-5-sulfonique et/ou du 2-hydroxybenzoate de 2-éthylhexyle (INCI : Ethylhexyl Salicylate).

8. Préparation, procédé ou utilisation selon l'une quelconque des revendications précédentes, caractérisé(e) en ce que la préparation contient du 2-[4-(diéthylamino)-2-hydroxybenzoyl]benzoate d'hexyle (INCI : Diethylamino hydroxybenzoyl hexyl benzoate) en une concentration de 0,05 à 1,5 % en poids, par rapport au poids total de la préparation.

9. Préparation, procédé ou utilisation selon l'une quelconque des revendications précédentes, caractérisé(e) en ce que la préparation contient du 4-(tert-butyl)-4'-méthoxydibenzoylméthane (INCI Butyl Methoxydibenzoylmethane) en une concentration de 1,00 à 5,00 % en poids, par rapport au poids total de la préparation.

10. Préparation, procédé ou utilisation selon l'une quelconque des revendications précédentes, caractérisé(e) en ce que le rapport pondéral de Diethylamino hydroxybenzoyl hexyl benzoate à Butyl Methoxydibenzoylmethane est de 0,2:1 à 1:5.

11. Préparation, procédé ou utilisation selon l'une quelconque des revendications précédentes, caractérisé(e) en ce que la préparation contient du stéarate de polyglycéryle-10 (INCI Polyglyceryl-10 Stearate) en une concentration de 0,05 à 1,25 % en poids, par rapport au poids total de la préparation.

12. Préparation, procédé ou utilisation selon l'une quelconque des revendications précédentes, caractérisé(e) en ce que la concentration totale en 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine) et en 2,4,6-tris-[anilino-(p-carbo-2'-éthyl-1 hexyloxy)]-1,3,5-triazine (INCI: Ethylhexyl Triazone) dans la préparation est de 0,05 à 4,00 % en poids, par rapport au poids total de la préparation.

13. Préparation, procédé ou utilisation selon l'une quelconque des revendications précédentes, caractérisé(e) en ce que la concentration totale en sels d'acide 2-phénylbenzimidazole-5-sulfonique et en 2-hydroxybenzoate de 2-éthylhexyle (INCI : Ethylhexyl Salicylate) dans la préparation est de 0,05 à 5,00 % en poids, par rapport au poids total de la préparation.

14. Préparation, procédé ou utilisation selon l'une quelconque des revendications précédentes, caractérisé(e) en ce que la préparation contient une combinaison de phénoxyéthanol et d'acétate de tocophéryle ou de tocophérol.

15. Préparation, procédé ou utilisation selon l'une quelconque des revendications précédentes, caractérisé(e) en ce que la préparation contient un ou plusieurs des matières parfumées salicylate d'hexyle, acétate de linayle, 2-isobutyl-4-hydroxy-4-méthyltétrahydropyranne, diester de l'acide adipique, méthylhepténone, alpha-isométhylionone, butylphénylméthylpropional, coumarine, hexylcinnamal, limonène, linalool, diéthylsuccinate, hydroxyisohexyl-3-cyclohexènecarboxaldéhyde, diéthylsuccinate, menthyle PCA et crotonate de citronellylméthyle, benzoate de benzyle, alpha-isométhylionone, alcool benzylique, cinnamate de benzyle, salicylate de benzyle, citronellol, eugénol, géraniol.

16. Préparation, procédé ou utilisation selon l'une quelconque des revendications précédentes, caractérisé(e) en ce que la préparation contient de l'éthylhexylglycérine, du propylèneglycol, du butylèneglycol, du 2-méthylepropane-1,3-diol, du 1,2-pentanediol, du 1,2-hexanediol, du 1,2-octanediol et/ou du 1,2-décanediol.

17. Préparation, procédé ou utilisation selon l'une quelconque des revendications précédentes, caractérisé(e) en ce que la préparation ne contient pas de parabène, ainsi que pas de méthylisothiazolinone, de chlorométhylisothiazolinone ni de DMDM-hydantoïne, c'est-à-dire est exempte de ces ingrédients.
